# FASCICULE DE BREVET EUROPEEN

(11) **EP 2 293 914 B1**
(45) Date de publication et mention de la délivrance du brevet: **14.03.2012**
(21) Numéro de dépôt: 08806125.4
(22) Date de dépôt: 30.06.2008
(51) Int. Cl.: B29C 45/00, A61L 31/06

(54) **Procede de moulage de poly(1,4-dioxanone)**
Verfahren zum Formen von Poly(1,4-Dioxanon)
Method for moulding poly(1,4-dioxanone)

(43) Date de publication de la demande: 16.03.2011
(73) Titulaire: CEREBEL-INVEST SA, 1001 Lausanne (CH)
(72) Inventeur: LE GOFF, Philippe, CH-1066 Epalinges (CH); ANDRIEU, Raymond, CH-1169 Yens (CH)
(74) Mandataire: Micheli & Cie SA
(86) Numéro de dépôt international: PCT/FR2008/051199
(87) Numéro de publication internationale: WO 2010/000954

(56) Documents cités:
- EP-A- 0 415 783
- EP-A- 1 591 487
- US-A- 4 444 927
- US-A- 4 490 326
- US-A- 5 234 449
- US-A- 5 869 597
- US-A1- 2008 132 604

## Description

La présente invention concerne un nouveau procédé de moulage de poly(1,4-dioxanone) en vue de la fabrication d'un dispositif médical biorésorbable.

Depuis près de 50 ans, l'intérêt du domaine médical pour des implants chirurgicaux à base de matériaux non permanents destinés à disparaître au cours du temps, n'a fait que croître.

Ces matériaux sont généralement qualifiés de « biodégradables », « bioérodables », « bioabsorbables » ou « biorésorbables ». Dans le cadre de la présente demande, nous utiliserons préférentiellement le terme « biorésorbable » ou « biodégradable ».

Dans le cas des implants biodégradables, la question de l'innocuité des produits de dégradation du matériau se pose dans la mesure où la totalité de la matière constitutive de l'implant ou du dispositif médical est libérée dans l'organisme du patient pour y être transformée et/ou métabolisée.

Parmi les matériaux polymères biodégradables, on distingue généralement les matériaux biologiques d'origine naturelle, tels que le collagène ou la cellulose, et les polymères de synthèse. La variété des polymères biodégradables disponibles et compatibles avec une utilisation médicale reste toutefois à ce jour encore relativement peu étendue ce qui limite plus ou moins directement la gamme des propriétés mécaniques des dispositifs médicaux susceptibles d'être fabriqués à partir de ceux-ci.

Au cours des deux dernières décennies, de très nombreuses structures polymériques potentiellement biodégradables par un processus d'hydrolyse ont été proposées sans qu'aucune n'ait atteint un stade de développement suffisant pour que l'on puisse envisager une utilisation dans le domaine des dispositifs médicaux.

Dans ce contexte, au début des années 2000, principalement cinq polymères étaient approuvés par la *Federal Drug Administration* (FDA), en vue de leur utilisation dans des implants. Ces cinq polymères sont le poly(acide lactique), le poly(acide glycolique), les poly(dioxanone), les poly(caprolactone) et les poly(anhydride). Bien entendu, les copolymères renfermant deux types de monomères, ou plus, constitutifs de ces homopolymères présentent une innocuité comparable à ceux-ci.

Les poly(dioxanone) et en particulier la poly(1,4-dioxanone) sont connues pour se dégrader par hydrolyse sans générer des produits de dégradation toxiques. La polydioxanone offre en outre l'avantage de se dégrader in vivo selon un processus d'hydrolyse seule, autrement dit la cinétique de dégradation de la polydioxanone n'est pas modifiée par des processus enzymatiques.

Cependant, les applications de dispositifs médicaux à base de poly(dioxanone) restent limitées car ce polymère est difficile à mettre en oeuvre et est réputé pour donner, après mise en forme, des matériaux aux propriétés mécaniques médiocres.

On connaît différents procédés de moulage par les documents US 4490326, US 5234449, US 4444927, US 5869597 et EP 1591487.

Le problème que se propose de résoudre l'invention est la réalisation d'une pièce moulée avec une haute cristallinité.

L'objet de la présente invention se distingue, notamment du document US 4490326 considéré comme l'état de la technique le plus proche par les caractéristiques essentielles a) et b) de la revendication indépendante 1.

De plus aucun des documents ci-dessus ne montre ni ne rend évident de telles caractéristiques; en particulier la haute température du moule n'est pas suggérée dans ces documents.

Le brevet US 4490326 propose un procédé de moulage par injection de polydioxanone en vue de la fabrication de dispositifs chirurgicaux implantables, biorésorbables, présentant une combinaison satisfaisante de propriétés mécaniques (résistance mécanique, ténacité, flexibilité, intégrité fonctionnelle). Ce document recommande le moulage par injection de la polydioxanone à partir d'une masse fondue ayant une température la plus proche possible de la température de fusion de la polydioxanone (P_{f} = 109 - 110° C). Ainsi, le brevet US 4490326 décrit que la polydioxanone préalablement fondue dans une extrudeuse, présentant une température comprise entre 110° C et 140° C, et de préférence entre 110° C et 115° C, est injectée dans un moule maintenu à une température au plus égale à 35° C, et est maintenue sous pression pendant un temps suffisant pour obtenir le durcissement total ou partiel de la pièce, avant d'être démoulée.

S'il est vrai qu'un tel procédé de moulage permet d'obtenir des pièces moulées présentant, au moment du démoulage, une combinaison de propriétés mécaniques plutôt satisfaisante, il importe également que ces propriétés perdurent pendant un temps suffisamment long, notamment au cours du stockage de la pièce moulée, pour pouvoir envisager une application à l'échelle industrielle. Or, la demanderesse a observé que les conditions de fabrication des pièces moulées influaient notablement sur ces propriétés mécaniques qui ne se maintenaient malheureusement pas au cours du temps. Au bout d'une période de stockage à température ambiante d'environ un mois, les pièces moulées devenaient cassantes et friables. Leur résistance mécanique devenue insuffisante interdisait alors de les utiliser en tant qu'implants biorésorbables. On comprendra aisément que cette non-pérennité des propriétés mécaniques des pièces moulées est un inconvénient considérable dans la perspective d'une commercialisation de dispositifs médicaux implantables à base de polydioxanone.

Dans le cadre de ses recherches visant à mettre au point de nouveaux dispositifs médicaux implantables à base de matériaux polymères biorésorbables, la Demanderesse a constaté avec surprise que, contrairement aux enseignements du brevet US 4 490 326, le moulage de polydioxanone à des températures supérieures à celles recommandées dans ce document permettait d'obtenir des pièces moulées qui non seulement présentaient des propriétés mécaniques satisfaisantes, mais qui conservaient avantageusement ces propriétés pendant plusieurs mois.

La Demanderesse a constaté par ailleurs que, pour obtenir les propriétés mécaniques intéressantes à partir d'une masse fondue de polydioxanone chauffée à une température relativement importante, c'est-à-dire de l'ordre de 145 °C à 165 °C, il était important de ne pas refroidir trop brusquement la masse fondue pour obtenir une bonne cristallinité de la polydioxanone.

La présente invention a par conséquent pour objet un procédé de moulage d'un polymère biorésorbable en vue de la fabrication d'un dispositif médical biorésorbable, comprenant les étapes successives suivantes :
(a) chauffage d'une poly(1,4-dioxanone), en l'absence d'un quelconque solvant de ce polymère, jusqu'à une température de masse comprise entre 145 °C et 165 °C,
(b) moulage par injection de la masse fondue, obtenue à l'étape (a), dans un moule qui se trouve à une température inférieure de 80 °C à 115 °C à la température de masse de la poly(1,4-dioxanone),
(c) refroidissement du moule jusqu'à solidification de la masse de poly(1,4-dioxanone), et
(d) démoulage de la pièce ainsi obtenue.

Le terme « température de masse » utilisé dans la présente invention désigne la température de la polydioxanone, mesurée à l'aide d'un thermomètre au centre de la masse fondue. Cette température de masse est inférieure à la température de consigne du dispositif de chauffage et également inférieure à la température du creuset servant à fondre le polymère.

Dans le cadre d'essais systématiques réalisés à différentes températures, la Demanderesse a constaté qu'il était possible de modifier les propriétés mécaniques des matériaux polymères moulés obtenus en choisissant de manière appropriée la température de masse pendant l'étape (a) : des températures comprises dans la moitié supérieure de la gamme revendiquée aboutissent ainsi à des matériaux polymères moulés plutôt rigides, alors que des températures de chauffage comprises dans la moitié inférieure de cette gamme donnent des matériaux plutôt souples.

Par conséquent, dans un mode de réalisation du procédé de l'invention, lorsqu'il s'agit d'obtenir des matériaux en poly(1,4-dioxanone) souples, on chauffe la poly(1,4-dioxanone) à l'étape (a) jusqu'à une température de masse comprise entre 145 °C et 155 °C.

Dans un autre mode de réalisation du procédé, lorsqu'il s'agit au contraire de préparer des matériaux relativement plus rigides, on chauffe la poly(1,4-dioxanone) à l'étape (a) jusqu'à une température de masse comprise entre 155 °C et 165 °C.

De façon générale, les matériaux moulés les plus intéressants sont ceux préparés à partir d'une masse fondue chauffée à une température de masse voisine de 155 °C, autrement dit la température de masse de la poly(1,4-dioxanone) à l'étape (a) est de préférence comprise entre 148 °C et 162 °C, en particulier entre 152 °C et 158 °C, et idéalement entre 154 °C et 156 °C.

Afin de prévenir d'éventuelles dégradations thermiques de la polydioxanone, il est souhaitable que l'étape de chauffage (étape (a)) du procédé de l'invention ait une durée relativement courte, de préférence d'autant plus limitée que la température de masse est élevée. De manière générale, la durée totale de l'étape de chauffage (a), comprenant la phase de montée en température et la phase de maintien de la température avant moulage, est inférieure à 60 minutes, de préférence inférieure à 45 minutes, en particulier compris entre 10 et 30 minutes.

La fusion de la polydioxanone peut être mise en oeuvre par exemple dans un creuset, placé sur une plaque chauffante électrique. Compte tenu de la viscosité élevée de la masse fondue de polydioxanone, cette étape de fusion est mise en oeuvre de préférence en l'absence d'agitation mécanique.

La poly(1,4-dioxanone) utilisée dans le procédé de la présente invention a de préférence une masse moléculaire relativement peu élevée qui est telle que sa viscosité inhérente, mesurée en solution à 0,1 % en poids dans l'hexafluoroisopropanol (HFIP) à une température de 30 °C est comprise entre 1,1 et 1,8 dl/g, et de préférence comprise entre 1,2 et 1,6 dl/g. Cette gamme de viscosité inhérente, et donc cette masse moléculaire du polymère, est préférée en raison des bonnes propriétés mécaniques conférées aux pièces moulées obtenues et du comportement compatible avec les contraintes thermiques et cinétiques du procédé de moulage de la présente invention.

Comme indiqué précédemment, la température du moule dans lequel est injectée la masse fondue de poly(1,4-dioxanone) est inférieure de 80 °C à 115 °C à celle de la température de masse du polymère fondu. Cette différence de température entre le polymère injecté et le moule garantit un bon aspect de la surface de la pièce obtenue et une cristallisation suffisante pour conférer à la pièce une résistance mécanique satisfaisante. Dans un mode de réalisation préféré du procédé de l'invention, la température du moule à l'étape (b) est inférieure de 85 °C à 105 °C, de préférence inférieure de 90 à 100 °C à la température de masse de la poly(1,4-dioxanone) de l'étape (a).

L'introduction du polymère fondu dans le moule peut se faire par exemple par injection du contenu de fusion dans le moule receveur. A ces fins, le fond du creuset est rendu mobile de façon à permettre l'injection de la matière fondue par un effet de piston provoqué par la pression exercé par le vérin d'une presse hydraulique sur la face externe du fond mobile du creuset. Le creuset, en combinaison avec un entonnoir approprié fixé sur le moule, agit ainsi comme une seringue d'injection de la matière fondue.

Il est fortement recommandé de maintenir la masse fondue, après injection dans le moule, pendant un certain temps sous la pression d'injection.

La durée du maintien sous pression de la masse fondue de polydioxanone dans le moule dépend de la taille et de la géométrie de la pièce, de la température du moule et de la vitesse de refroidissement de celui-ci au cours de l'étape (c). L'expérience montre qu'une durée comprise entre 5 secondes et 40 secondes convient et qu'une durée comprise entre 10 et 20 secondes est particulièrement avantageuse.

Après injection de la poly(1,4-dioxanone) fondue, le moule, initialement à la température indiquée précédemment, est refroidi lentement. Ce refroidissement peut se faire par simple arrêt du chauffage et dissipation de la chaleur ou bien par refroidissement actif du moule, par exemple par contact avec une surface froide.

La durée totale de la phase de refroidissement (étape (c)) est de préférence comprise entre 1 et 30 minutes, en particulier entre 2 et 10 minutes.

La pièce en poly(1,4-dioxanone) durcie n'est de préférence démoulée, à l'étape (d), que lorsque sa température de surface a atteint une valeur inférieure à 50 °C, de préférence comprise entre la température ambiante et 45 °C.

La présente invention a également pour objet une pièce moulée en poly(1,4-dioxanone) susceptible d'être obtenue par le procédé décrit ci-dessus. Les pièces en poly(1,4-dioxanone) obtenues selon le procédé de la présente invention ont en effet des propriétés différentes de celles des pièces moulées en polydioxanone, préparées selon l'art antérieur. Elles se caractérisent en particulier par une plus grande stabilité des propriétés mécaniques au cours du temps. Les pièces en poly(1,4-dioxanone) obtenues selon le procédé de la présente invention, ne deviennent pas cassantes au bout d'un mois seulement et peuvent être conservées, avant implantation, pendant au moins 12 mois, généralement pendant au moins 24 mois à partir du démoulage.

Enfin, la présente invention a également pour objet un dispositif médical constitué d'une telle pièce moulée en poly(1,4-dioxanone), fabriqué à partir de celle-ci par exemple par façonnage et/ou contenant au moins une telle pièce.

### Exemple

On introduit 3,0 g de poly(1,4-dioxanone) (viscosité inhérente 1,4 dl/g, à 30 °C dans HFIP) dans un creuset de fusion à fond mobile et on place le creuset contenant le polymère sur une plaque chauffante préalablement réglée à environ 220 °C. Un thermomètre est introduit au centre de la masse fondue. Parallèlement, un moule est conditionné à une température comprise entre 50 et 60 °C.

Lorsque la température de masse de la poly(dioxanone) mesurée à l'aide du thermomètre atteint environ 148 à 152 °C, et en veillant à ce que la durée de chauffage n'excède pas 30 minutes, on place le creuset en position renversée sur l'entonnoir du moule et on actionne le vérin d'une presse hydraulique de manière à injecter la polydioxanone fondue dans le moule. La pression du vérin sur le fond mobile du creuset est maintenue pendant environ 15 secondes.

Le vérin est ensuite relevé et le creuset désaccouplé du moule. On refroidit le moule en le plaçant pendant environ 5 minutes sur une plaque maintenue à la température ambiante. On ouvre ensuite le moule et l'on extrait la pièce moulée à l'aide d'une pince Bruxelles.

## Revendications

1. Procédé de moulage d'un polymère biorésorbable en vue de la fabrication d'un dispositif médical biorésorbable, comprenant les étapes successives suivantes :
(a) chauffage d'une poly(1,4-dioxanone), en l'absence d'un quelconque solvant de ce polymère, jusqu'à une température de masse comprise entre 145 °C et 165 °C,
(b) moulage par injection de la masse fondue, obtenue à l'étape (a), dans un moule qui se trouve à une température inférieure de 80 °C à 115 °C à la température de masse de la poly(1,4-dioxanone),
(c) refroidissement du moule jusqu'à solidification de la masse de poly(1,4-dioxanone), et
(d) démoulage de la pièce ainsi obtenue.

2. Procédé de moulage selon la revendication 1, **caractérisé par le fait que** l'on chauffe la poly(1,4-dioxanone) à l'étape (a) jusqu'à une température de masse comprise entre 148 °C et 162 °C, de préférence entre 152 °C et 158 °C, et en particulier entre 154 °C et 156 °C.

3. Procédé de moulage selon la revendication 1, **caractérisé par le fait que** l'on chauffe la poly(1,4-dioxanone) à l'étape (a) jusqu'à une température de masse comprise entre 145 °C et 155 °C.

4. Procédé de moulage selon la revendication 1, **caractérisé par le fait que** l'on chauffe la poly(1,4-dioxanone) à l'étape (a) jusqu'à une température de masse comprise entre 155 °C et 165 °C.

5. Procédé de moulage selon l'une quelconque des revendications précédentes, **caractérisé par le fait que** la température du moule à l'étape (b) est inférieure de 85 °C à 105 °C, de préférence inférieure de 90 à 100 °C à la température de masse de la poly(1,4-dioxanone) de l'étape (a).

6. Procédé de moulage selon l'une quelconque des revendications précédentes, **caractérisé par le fait que** la poly(1,4-dioxanone) a une masse moléculaire telle que sa viscosité inhérente, mesurée en solution à 0,1 % en poids dans l'hexafluoroisopropanol (HFIP) à une température de 30 °C, est comprise entre 1,1 et 1,8 dl/g, et de préférence entre 1,2 et 1,6 dl/g.

7. Procédé de moulage selon l'une quelconque des revendications précédentes, **caractérisé par le fait que** la durée totale de l'étape de chauffage (a) est inférieure à 60 minutes, de préférence inférieure à 45 minutes, en particulier compris entre 10 et 30 minutes.

8. Procédé de moulage selon l'une quelconque des revendications précédentes, **caractérisé par le fait que** la durée de l'étape de refroidissement (étape (c)) est comprise entre 1 et 30 minutes, de préférence entre 2 et 10 minutes.

9. Procédé de moulage selon l'une quelconque des revendications précédentes, **caractérisé par le fait que** la pièce en poly(1,4-dioxanone) est démoulée (étape (d)) lorsque sa température de surface de la pièce est inférieure à 50 °C, de préférence comprise entre la température ambiante et 45 °C.

## Claims

1. Method of moulding a bioresorbable polymer for producing a bioresorbable medical device, comprising the following successive steps:
(a) heating a poly(1,4-dioxanone) in the absence of any solvent of this polymer to a mass temperature between 145°C and 165°C,
(b) injection moulding of the molten mass obtained in step (a) in a mould which is at a temperature of 80°C to 115°C lower than the mass temperature of the poly(1,4- dioxanone),
(c) cooling the mould until solidification of the mass of poly(1,4-dioxanone), and
(d) removing the thus obtained part from the mould.

2. Moulding method as claimed in Claim 1, **characterised in that** the poly(1,4-dioxanone) is heated in step (a) to a mass temperature between 148°C and 162°C, preferably between 152°C and 158°C, and in particular between 154°C and 156°C.

3. Moulding method as claimed in Claim 1, **characterised in that** the poly(1,4-dioxanone) is heated in step (a) to a mass temperature between 145°C and 155°C.

4. Moulding method as claimed in Claim 1, **characterised in that** the poly(1,4-dioxanone) is heated in step (a) to a mass temperature between 155°C and 165°C.

5. Moulding method as claimed in any one of the preceding Claims, **characterised in that** the temperature of the mould in step (b) is 85°C to 105°C lower, preferably 90 to 100°C lower, than the mass temperature of the poly(1,4-dioxanone) of step (a).

6. Moulding method as claimed in any one of the preceding Claims, **characterised in that** the poly(1,4-dioxanone) has a molecular mass such that its inherent viscosity, measured in 0.1 wt.% solution in hexafluoroisopropanol (HFIP) at a temperature of 30°C, is between 1.1 and 1.8 dl/g and is preferably between 1.2 and 1.6 dl/g.

7. Moulding method as claimed in any one of the preceding Claims, **characterised in that** the total duration of heating step (a) is lower than 60 minutes, preferably lower than 45 minutes, in particular between 10 and 30 minutes.

8. Moulding method as claimed in any one of the preceding Claims, **characterised in that** the duration of the cooling step (step (c)) is between 1 and 30 minutes, preferably between 2 and 10 minutes.

9. Moulding method as claimed in any one of the preceding Claims, **characterised in that** the poly(1,4-dioxanone) part is removed from the mould (step (d)) when its surface temperature of the part is lower than 50°C, preferably between ambient temperature and 45°C.

## Patentansprüche

1. Verfahren zum Formen eines bioresorbierbaren Polymers zur Herstellung einer bioresorbierbaren medizinischen Vorrichtung, wobei das Verfahren die folgenden aufeinander folgenden Schritte umfasst :
(a) Erhitzen eines Poly-(1,4-dioxanons) in Abwesenheit von jeglichem Lösungsmittel dieses Polymers auf eine Massentemperatur zwischen 145 °C und 165 °C,
(b) Spritzgießen der geschmolzenen Masse, die in Schritt (a) erhalten wurde, in einer Form, die eine Temperatur aufweist, die 80 °C bis 115 °C unter der Temperatur der Masse des Poly-(1,4-dioxanons) liegt,
(c) Kühlen der Form bis zur Erstarrung der Masse des Poly-(1,4-dioxanons) und
(d) Ausformen des so erhaltenen Teils.

2. Formverfahren nach Anspruch 1, **dadurch gekennzeichnet, dass** das Poly-(1,4-dioxanon) in Schritt (a) auf eine Massentemperatur zwischen 148 °C und 162 °C, vorzugsweise zwischen 152 °C und 158 °C und insbesondere zwischen 154 °C und 156 °C erhitzt wird.

3. Formverfahren nach Anspruch 1, **dadurch gekennzeichnet, dass** das Poly-(1,4-dioxanon) in Schritt (a) auf eine Massentemperatur zwischen 145 °C und 155 °C erhitzt wird.

4. Formverfahren nach Anspruch 1, **dadurch gekennzeichnet, dass** das Poly-(1,4-dioxanon) in Schritt (a) auf eine Massentemperatur zwischen 155 °C und 165 °C erhitzt wird.

5. Formverfahren nach einem der vorhergehenden Ansprüche, **dadurch gekennzeichnet, dass** die Temperatur der Form in Schritt (b) 85 °C bis 105 °C, vorzugsweise 90 bis 100 °C unter der Temperatur der Masse des Poly-(1,4-dioxanons) in Schritt (a) liegt.

6. Formverfahren nach einem der vorhergehenden Ansprüche, **dadurch gekennzeichnet, dass** das Poly-(1,4-dioxanon) ein derartiges Molekulargewicht aufweist, dass seine Eigenviskosität, die in einer Lösung von 0,1 Gew.-% in Hexafluorisopropanol (HFIP) bei einer Temperatur von 30 °C gemessen wird, zwischen 1,1 und 1,8 dl/g und vorzugsweise zwischen 1,2 und 1,6 dl/g liegt.

7. Formverfahren nach einem der vorhergehenden Ansprüche, **dadurch gekennzeichnet, dass** die Gesamtdauer des Erhitzungsschritts (a) weniger als 60 Minuten, vorzugsweise weniger als 45 Minuten und insbesondere zwischen 10 und 30 Minuten ausmacht.

8. Formverfahren nach einem der vorhergehenden Ansprüche, **dadurch gekennzeichnet, dass** die Gesamtdauer des Kühlungsschritts (Schritt (c)) zwischen 1 und 30 Minuten, vorzugsweise zwischen 2 und 10 Minuten ausmacht.

9. Formverfahren nach einem der vorhergehenden Ansprüche, **dadurch gekennzeichnet, dass** das Teil aus Poly-(1,4-dioxanon) ausgeformt wird (Schritt (d)), wenn die Oberflächentemperatur des Teils unter 50 °C, vorzugsweise zwischen Umgebungstemperatur und 45 °C liegt.
